# EUROPEAN PATENT APPLICATION

(11) **EP 1 764 352 A1**
(43) Date of publication of application: **21.03.2007**
(21) Application number: 05023701.5
(22) Date of filing: 17.08.2005
(51) Int. Cl.: C07B 59/00, C07C 269/06, C07C 271/22, A61K 51/04

(54) **A conventional method for the preparation of new precursor of no-carrier-adde O-(2-[18F]fluoroethyl)-L-tyrosine**

(62) Divisional of application: 05017898.7
(71) Applicant: Institute of Nuclear Energy Research, Lungtan Taoyuan (TW)
(72) Inventor: Wang, Hsin Er c/o Inst.Nuclear Energy Research, Jiaan Village, Longtan, Taoyuan 32546 (CN); Wu, Shih Yan c/o Inst.Nuclear Energy Research, Jiaan Village,Longtan, Taoyuan 32546 (CN); Lin, Wuu Jyh c/o Inst.Nuclear Energy Research, Jiaan Village, Longtan, Taoyuan 32546 (CN); Lo, Ai Ren c/o Inst.Nuclear Energy Research, Jiaan Village, Longtan, Taoyuan County 3 (CN); Chen, Jenn Tzong c/o Inst. Nuclear Energy Research, Jiaan Village, Longtan, Taoyuan 32546 (CN); Li, Ming Hsin c/o Inst.Nuclear Energy, Jiaan Village, Longtan, Taoyuan 32546 (CN)
(74) Representative: Kador & Partner

(57) **Abstract**

This is a new precursor and new method for the synthesis of no-carrier-added *O*-(2-[¹⁸F]fluoroethyl)-L-Tyrosine which has been proved a suitable PET (position emission tomography) probe for tumor diagnosis imaging.

The preparation of the title compound starts from precursors with the chemical structures as in Formula 1, wherein R¹ is a protective group for the carboxyl functional group, R² is a protective group for the amino group, and R³ acts as a leaving group. R¹ represents an arylalkyl group, R² represents a carboxyl group, and R³ represents a *p*-tosyloxy, methane sulfonyloxy or trifluoromethane sulfonyloxy or bromine. The invention includes a method for the syntheses of new precursors with the chemical structures as in Formula 1. 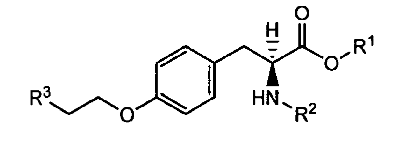 Formula 1 : Synthesis precursors for *O*-(2-[¹⁸F]fluoroethyl)-L-Tyrosine.

## Description

### Background of the invention

¹⁸F-labeled *O*-(2-[¹⁸F]fluoroethyl)-L-Tyrosine is an amino acid tracer, it has been proved as a suitable PET (position emission tomography) probe for tumor diagnosis imaging.

The preparation of *O*-(2-[¹⁸F]fluoroethyl)-L-Tyrosine was developed by Wester et al. (J. Nucl. Med. 1999;40:205-212) and Hamacher et al. (Appl. Radiat. Isot. 2002;57:853-856). However, it is inconvenient by using high performance liquid chromatography (HPLC) for purified product, it is not only cumbersome but also difficult automation, since during HPLC purify process operator must switch valve from waste collecting bottle to product bottle in order to collect purified *O*-(2-[¹⁸F]fluoroethyl)-L-Tyrosine and switch valve back to waste bottle after complete the collection of purified product in order to maintain high purified *O*-(2-[¹⁸F]fluoroethyl)-L-Tyrosine. The present invention provides a new precursor for smooth synthesis, and utilizes resin and silica gel column for purification to reduce complexity of HPLC purification process.

### Cited references

1. H.J.Wester, M.Herz, W.Weber, P.Heiss, R.Senekowitsch-Schmidtke, M.Schwaiger and G.Stöcklin, Synthesis and radiopharmacology of O-(2-[18F]fluoroethyl)-L-Tyrosine for tumor imaging. J. Nucl. Med. 40, 205-212 (1999).
2. K.Hamacher and H.H.Coenen, Efficient routine production of the 18F-labelled amino acid O-(2-[18F]fluoroethyl)-L-Tyrosine, Appl. Radiat. Isot. 57, 853-856 (2002).

### Summary of the invention

The object of this invention provides a synthetic method of novel t-BOC-(*O*-tosyloxyethyl)-L-Tyr-OBzl (as Formula 1), which is a precursor of *O*-(2-[¹⁸F]fluoroethyl)-L-Tyrosine.

For the synthesis of *O*-(2-[¹⁸F]fluoroethyl)-L-Tyrosine, the initial labeled-compound (that is precursor) is prepared above all. This synthesis procedure is carried out in chemistry lab instead of in radiation control area because of no radiation.

### Precursor of O-(2-[¹⁸F]fluoroethyl)-L-Tyrosine

Synthesis of *O*-(2-[¹⁸F]fluoroethyl)-L-Tyrosine : (1) Prepare ethylene glycol-1,2-ditosylate. React ethylene glycol and toluenesulfonyl chloride in pyridine solution at low temperature for two to three days, warming the solution, solidify the product and purify by re-crystallization to obtain ethylene glycol-1,2-ditosylate. (2) Prepare
t-Boc-(*O*-tosyloxyethyl)-L-Tyr-Obzl. Add t-Boc-L-Tyr-OBzl in acrylonitrile solution contains ethylene glycol-1,2-ditosylate and potassium carbonate, heat to 90°C in stirring for react four hours, remove solvent after complete the reaction, then extract with chloroform to obtain solid residue, purify dissolved residue by column chromatography and obtain pure t-Boc-(*O*-tosyloxyethyl)-L-Tyr-OBzl product.

### Detailed description of the invention

The embodiments of the invention will be described as follows:
**The synthetic method of ¹⁸F-labeled precursor**
**t-BOC-(*O*-tosyloxyethyl)-L-Tyr-OBzl**

### 1. Preparation of ethylene glycol-1,2-ditosylate

(1) Add 17g of toluenesulfonyl chloride (TsCl)(F.W.=190.65, 0.089 mol) into conical flask (A) with 20 ml of pyridine.
(2) Add 1.1 ml of ethylene glycol (F.W.=62.07 , 0.018 mol) into conical flask (B) with 30 ml of pyridine.
(3) Pour solution of conical flask (A) into conical flask (B) under the temperature of dry ice-acetone bath (-30°C approximately) and then put the flask under -18°C promptly for react two to three days.
(4) After complete reaction, pour the reactant in conical flask (B) into 500 ml beaker with ice water and cracked ice; white solid substance will appear after stirring.
(5) Add optimal 1N HCl into beaker above-mentioned and adjust pH to 6-7.
(6) Filter and collect white solid substance, then re-crystallize in mixture of methylene chloride and normal hexane to yield 80% of 5.33g ethylene glycol-1,2-ditosylate.

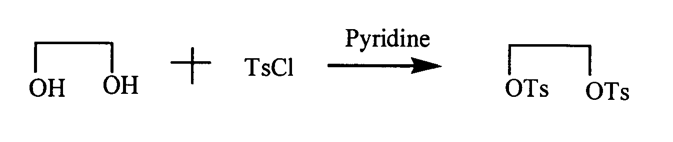

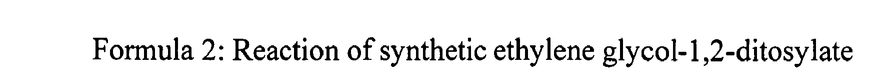

### 2. Preparation of t-BOC-(O-tosyloxyethyl)-L-Tyr-OBzl

Following is the synthetic process of using t-BOC-L-Tyr-OBzl as a raw material (Formula 2): 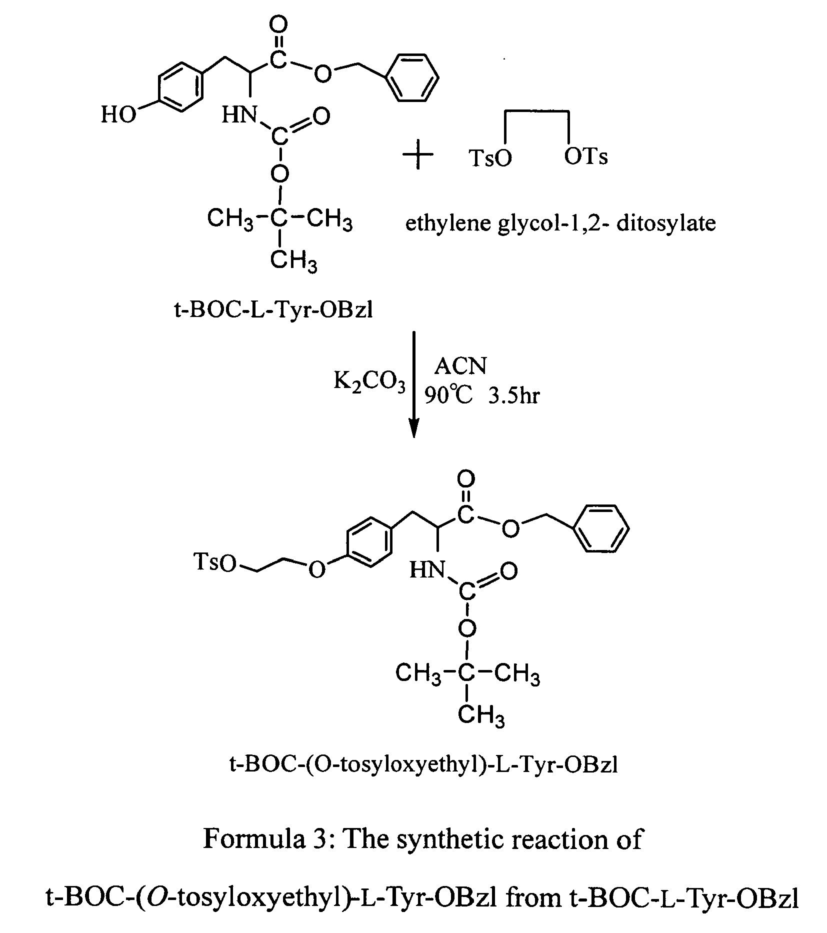
(1) Add 450mg of *N-tert*-butyloxycarbonyl- L-tyrosine benzylester (t-BOC-L-Tyr-OBzl) (F.W.=361, 1.24 mmol) into 50ml of round bottomed flask contains 20mg potassium carbonate and 1.384g ethylene glycol-1,2- ditosylate (F.W.=370.35 , 3.73 mmol); then add 25ml anhydrous acetonitrile at 90°C and stir for 3.5 hours.
(2) After complete the reaction, remove solvent with rotavapor and extract with chloroform (5ml x3). Harvest chloroform extract and remove solvent under negative pressure.
(3) Dissolve solid residue with minimum amount of methylene chloride, then perform silica gel chromatography (add 0.1 % triethylamine in eluent) for purification. The initial condition of mobile phase is 100% CH₂Cl₂; after remove un-react ethylene glycol-1,2-ditosylate the condition of mobile phase change to CH₂Cl₂/CHCl₃=1/1, the crude product then eluted. Dry crude product under reduced pressure and obtain the solid crude product.
(4) Dissolve crude product with minimum volume solution of CH₂Cl₂:CHCl₃=8/2, then perform silica gel chromatography (add 0.1% triethylamine in eluent) for purification; the initial condition of mobile phase is CH₂Cl₂/CHCl₃=8/2 (another 0.1% triethyl amine is added); light yellow oil-like substance (398 mg) of pure *N-tert*-butyloxycarbonyl-(*O*-tosyloxyethyl)-L-tyrosine benzylester (t-BOC-(*O*-tosyloxyethyl)-L-Tyr-OBzl) is eluted; re-crystallize in dichloromethane and n- hexane to yield 60.1 % of white solid substance, melt point 85~86°C.
(5) Nuclear Magnetic Resonance (NMR): Dissolve 20mg of t-BOC-(*O*-tosyloxyethyl)-L-Tyr-OBzl in 0.6ml CDCl₃, then determine its ¹H-NMR spectrum (Formula 3). ¹H NMR (CDCl₃) δ 7.80 (d, 2H, J=8.4 Hz, Haryl), 7.31 (m, 7H, Haryl), 6.89 (d, 2H, J=8.4 Hz, Haryl), 6.62 (d, 2H, J=8.4 Hz, Haryl), 5.15 (d, 1H, 12.2 Hz, CH of benzyl), 5.08 (d, 1H, 12.2 Hz, CH of benzyl), 4.92 (d, 1H, J=8.0Hz, NH), 4.54 (m, 1H, CH), 4.33 (t, 2H, J=4.6 Hz, CH₂), 4.07 (t, 2H, J=4.6 Hz, CH₂), 2.99 (d, 2H, J=5.8 Hz, CH₂ of Tyr), 2.43 (s, 3H, CH₃ of toluene), 1.39 (s, 9H, CH₃ of t-BOC).
(6) Elemental analysis: The formula of
   t-BOC-(*O*-tosyloxyethyl)-L-Tyr-OBzl is C₃₀H₃₅NO₈S, the calculated value of elemental analysis is: C, 63.27; H, 6.15; N, 2.46. The actual value is: C, 63.34; H, 5.62; N, 2.33.

## Claims

1. A method for produce a precursor of ¹⁸F-labeled *O*-(2-[¹⁸F]fluoroethyl)-L-Tyrosine, which has the following chemical structure: 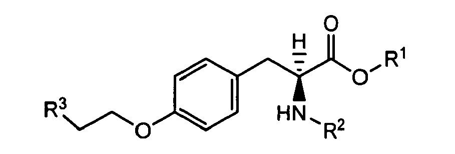 wherein R¹ is a protective group for the carboxyl functional group and represents an arylalkyl group;
R² is a protective group for the amino group and represents a carboxyl group; and
R³ acts as a leaving group and represents a *p*-tosyloxy, methane sulfonyloxy or trifluoromethane sulfonyloxy or bromine.
